# EUROPÄISCHE PATENTANMELDUNG

(11) **EP 1 454 639 A1**
(43) Veröffentlichungstag der Anmeldung: **08.09.2004**
(21) Anmeldenummer: 04000579.5
(22) Anmeldetag: 14.01.2004
(51) Int. Cl.: A61L 2/20, B65B 55/10

(54) **Sterilisationsverfahren in einem Verpackung und Transportsystem**

(30) Priorität: 27.02.2003 DE 10310000; 08.09.2003 DE 10341978
(71) Anmelder: Rüdiger Haaga GmbH, 78727 Oberndorf (DE)
(72) Erfinder: Frost, Robert Dr., 84034 Landshut (DE)
(74) Vertreter: Wilhelm, Martin

(57) **Zusammenfassung**

Beschrieben wird ein Verfahren zum Handhaben eines Verpackungs-Transportsystems, bei welchem kontaminierte Gegenstände in einer kontaminierten Verpackung keimdicht, aber gasdurchlässig verpackt und in diesem Zustand, beispielsweise durch ein diffusionsfreudiges Gas, sterilisiert werden und die Verpackung in diesem Zustand, gegebenenfalls nach Entfernen einiger Verpackungsteile, in einer Schleuse auf ihrer Außenseite erneut sterilisiert und in einen sterilen Reinraum eingeführt wird. Bei den Gegenständen handelt es sich insbesondere um mit Injektionsnadeln versehene Spritzenkörper, die in einer im Reinraum befindlichen Füllvorrichtung befüllt und verschlossen werden sollen. Das erneute Sterilisieren der Außenseite der Verpackung, beispielsweise von die Spritzenkörper enthaltenden, einer zusätzlichen Umverpackung entledigten Transportbehältern, geschieht in einer als Schleuse dienenden evakuierbaren Sterilisationskammer. Dabei wird ein aus Wasserdampf und Wasserstoffperoxiddampf bestehendes Dampfgemisch durch Vorevakuieren der Sterilisationskammer schlagartig als Kondensatbelag auf die Außenseite der Transportbehälter aufgebracht, und unmittelbar danach werden der Kondensatbelag und das nicht kondensierte Dampfgemisch durch weiteres Evakuieren aus der Sterilisationskammer entfernt. Dies geschieht in einer Weise, dass die verpackten Gegenstände dabei nicht beeinträchtigt werden.

## Beschreibung

Die Erfindung betrifft ein Verfahren zum Handhaben eines Verpackungs-Transportsystems, bei welchem kontaminierte Gegenstände in einer kontaminierten Verpackung keimdicht, aber gasdurchlässig verpackt und in diesem Zustand sterilisiert werden und die Verpackung in diesem Zustand, gegebenenfalls nach Entfernen einiger Verpackungsteile, in einer Schleuse auf ihrer Außenseite erneut sterilisiert und in einen sterilen Reinraum eingeführt wird.

Die Erfindung betrifft insbesondere ein Verfahren zum rekontaminationsfreien Einführen von bereits sterilisierten, gegebenenfalls mit Injektionsnadeln versehenen Spritzenkörpern in eine von einem sterilen Reinraum umgebene Füllvorrichtung zum Befüllen und Verschließen der Spritzenkörper, bei welchem Spritzenkörper enthaltende sterilisierte Transportbehälter (so genannte Tubs), die mit einer keimdichten; aber gasdurchlässigen Abdeckung versehen und zusätzlich von einer ebenfalls keimdichten und gasdurchlässigen Umverpackung umschlossen sind, zunächst von der Umverpackung befreit und danach, während sie noch mit der Abdeckung versehen sind, auf ihrer Außenseite erneut sterilisiert und in den Reinraum eingeführt werden.

Ein Verfahren der letztgenannten Art, für das der Anmelderin keine druckschriftliche Veröffentlichung bekannt ist, ist durch die Praxis Stand der Technik. Die erwähnten Transportbehälter oder Tubs sind Kunststoffwannen, in die ein gelochtes Tablett, häufig Nest genannt, eingesetzt ist. In den Löchern hängen fertig produzierte Spritzenkörper, die gegebenenfalls bereits mit eingeklebten Nadeln und aufgesetzten Nadelverschlusskappen sowie eventuell mit Nadelschutzkappen versehen sind. Diese Fertigspritzen sind bereits sterilisiert worden und brauchen nur noch befüllt und mit einem Druckkolben verschlossen zu werden. Die Kunststoffwannen sind auf ihrer Oberseite mit einer zwar keimdichten, aber gasdurchlässigen Folie versiegelt. Diese besteht in der Regel aus Polyester und ist durch den Markennamen Tyvek bekannt. Eine derartige Folie besitzt Poren, die so groß sind, dass Gasmoleküle hindurch diffundieren können, die aber andererseits so klein sind, dass Mikroorganismen nicht hindurchdringen können.

Die Tubs werden mit einer beutelartigen Umverpackung versehen, in welche ebenfalls ein keimdichter, aber gasdurchlässiger Tyvek-Streifen integriert ist. Anschließend werden die umverpackten Tubs in aller Regel mit Ethylenoxid, einem hochgiftigen Gas, sterilisiert. Dieses Gas dringt bei der Sterilisation sowohl durch die Umverpackung als auch durch die Abdeckung der Kunststoffwanne ins Innere der Tubs vor und sterilisiert dabei sämtliche Flächen innerhalb der Umverpackung. Danach wird das Ethylenoxid durch Evakuieren wieder abgezogen, und nach einer mehrtägigen Ausgasphase in einem speziellen Lagerraum sind die Tubs verkaufsbereit. Alternativ ist es bekannt geworden, solche Tubs durch Gamma-Bestrahlung zu sterilisieren.

Zur Befüllung der Spritzenkörper und deren Verschließen mittels des Druckkolbens müssen die Spritzenkörper in eine Füllvorrichtung eingeführt werden. Das Abfüllen flüssiger empfindlicher Pharmazeutika erfolgt stets unter sterilen Bedingungen. Die Abfüllvorrichtungen sind entweder in entsprechenden Reinräumen installiert oder besitzen einen eigenen kleinen Reinraum, mit dem sie überbaut sind, einen so genannten Isolator.

Die Schwierigkeit besteht nun darin, die Spritzenkörper ohne Rekontamination in absolut sterilem Zustand in den Reinraum einzuführen. Selbstverständlich dürfen dabei keine Keime in den Reinraum selbst eingeführt werden.

Bei dem bekannten Verfahren wird zunächst außerhalb des Reinraumes die durch Abwischen mit einem alkoholgetränkten Tuch desinfizierte Umverpackung, beispielsweise mittels mit Alkohol desinfizierter Handschuhe, entfernt. Bereits da besteht die Gefahr, dass keimbesetzte Partikel von der Umverpackung auf das Tub fallen. Nach anschließendem Einschleusen in den Reinraum wird die keimdichte Tyvek-Folie vom Tub abgezogen, beispielsweise über Eingriff-Handschuhe aus Hypalon, so dass man Zugriff zu den zu befüllenden Spritzenkörpern erhält. Falls jedoch die Tyvek- Folie mit keimbesetzten Partikeln rekontaminiert sein sollte, könnten diese Partikel beim Abziehen der Folie herunterfallen und an die Spritzenkörper gelangen. Um dies zu verhindern, liegt über den offenen Spritzenkörpern zusätzlich lose ein Blatt Tyvek, welches zuletzt manuell abgehoben wird, bevor das Nest in den Füller eingesetzt wird.

Um die genannten Gefahren zu minimieren, ist es seit einiger Zeit bekannt geworden, das der Umverpackung entledigte, aber noch mit der Tyvek-Abdeckung versiegelte Tub erneut zu sterilisieren, wünschenswert allerdings nur auf der Außenseite, damit die bereits sterilisierten Spritzenkörper in keiner Weise beeinträchtigt werden. Dieses Sterilisieren der Außenseite geschieht beispielsweise in einer Art Schleuse, in welcher das Tub mit Hochenergie-Elektronenstrahlen (E-Beam) sterilisiert wird. Dieses bisher einzige brauchbare Nachsterilisationsverfahren besitzt jedoch eine Reihe von Nachteilen: Es ist nicht nur kostspielig und technisch sehr aufwendig, sondern es sind auch Strahlenschutzmaßnahmen erforderlich. Dies erfordert Genehmigungen für den Aufbau und Betrieb sowie qualifiziertes Spezialpersonal. Außerdem werden durch die hochenergetische ionisierende Strahlung aggressive Radikale in großer Menge erzeugt, die an die Innenflächen der Spritzenkörper gelangen und nach dem Abfüllen mit dem Produkt reagieren können. Es bleibt somit bisher ein nicht erfüllter Wunsch, ausschließlich auf der Außenseite erneut zu sterilisieren.

Der Erfindung liegt nun die Aufgabe zu Grunde, eine technisch weit einfachere, kleinere und billigere Schleuse zu bauen, die ein Nachsterilisieren der Außenseite der Verpackung, insbesondere der von der Umverpackung entledigten Tubs möglich macht und auf die Verwendung der höchst gesundheitsschädlichen ionisierenden Strahlung verzichtet. Dabei soll auch sichergestellt sein, dass die Oberflächen der bereits sterilisierten Spritzenkörper, die mit dem abzufüllenden Produkt in Kontakt kommen, nicht mit irgendwelchen Radikalen belastet oder sonstwie beim Nachsterilisieren beeinträchtigt werden.

Die Aufgabe wird dadurch gelöst, dass das erneute Sterilisieren der Außenseite der Verpackung, insbesondere der Transportbehälter in einer als Schleuse dienenden evakuierbaren Sterilisationskammer durchgeführt wird, wobei ein aus Wasserdampf und Wasserstoffperoxiddampf bestehendes Dampfgemisch durch Vorevakuieren der Sterilisationskammer schlagartig als Kondensatbelag auf der Außenseite der Verpackung bzw. der Transportbehälter aufgebracht wird und unmittelbar danach der Kondensatbelag und das nicht kondensierte Dampfgemisch durch weiteres Evakuieren aus der Sterilisationskammer entfernt werden, so dass weder das Dampfgemisch noch der Kondensatbelag in unzulässiger Menge durch die Verpackung bzw. die Abdeckung hindurch zu den Gegenständen bzw. den Spritzenkörpern gelangen.

Bei dem Verfahren nach der Erfindung ist nun zunächst vorgesehen, die Sterilisationskammer mit einem Pumpstand vorzuevakuieren, damit der Druck in der Sterilisationskammer sinkt. Sobald der Vorevakuierungsdruck in der Sterilisationskammer erreicht ist, lässt man ohne jeglichen Trägergasstrom, ausschließlich getrieben durch die Druckdifferenz zwischen einem Verdampfer und der vorevakuierten Sterilisationskammer, ein aus Wasserdampf und Wasserstoffperoxiddampf bestehendes Dampfgemisch schlagartig in die Sterilisationskammer einströmen. Durch die dabei erfolgende Expansion des Dampfgemisches, welches nun nicht mehr lediglich das Volumen des Verdampfers, sondern auch das weitaus größere Volumen der Sterilisationskammer ausfüllt, kühlt dieses ab. Die Abkühlung führt zu einer starken Übersättigung des Dampfgemisches, weshalb dieses nun direkt im Moment des Einströmens auf allen ihm zugänglichen Oberflächen innerhalb der Sterilisationskammer kondensiert. Bei dieser Kondensation wird durch die frei werdende Verdampfungsenthalpie das entstehende Kondensat augenblicklich derart erhitzt, dass Wasserstoffperoxid in großer Menge dissoziiert, wobei die Mikroorganismen dabei praktisch schlagartig im Augenblick der Kondensation des in die Sterilisationskammer hinein expandierenden Dampfgemisches abgetötet werden, bevor das Dampfgemisch oder der Kondensatbelag in ins Gewicht fallender Weise die Abdeckung durchdringen können.

Die Geschwindigkeit des Vorevakuierens muss auf den Strömungswiderstand der Abdeckung der Transportbehälter abgestimmt werden. Dadurch lässt sich bei einer Variante des erfindungsgemäßen Verfahrens erreichen, dass der Druck innerhalb der abgedeckten Transportbehälter relativ zum Druck der Steritisationskammer zumindest anfangs höher ist, so dass das Dampfgemisch nicht durch die Abdeckung hindurch in das Innere der Transportbehälter und etwa gar an die Spritzenkörper gelangt. Es soll ja verhindert werden, dass auf der Außenseite der Abdeckung gebildetes Kondensat durch die gasdurchlässige Folie hindurch in die Tubs hinein gedrückt wird. Die einzelnen Parameter, nämlich der Druck im Verdampfer vor dem Einströmen, das Volumenverhältnis zwischen Verdampfer zu Sterilisationskammer, der Vorevakuierungsdruck sowie der Druck innerhalb der Tubs im Augenblick des Einströmens des Dampfgemisches müssen natürlich richtig aufeinander abgestimmt werden.

Da bei dem beschriebenen Verfahren die Abtötung der Keime instantan im Augenblick der Kondensatbildung erfolgt, kann das Entfernen des Kondensatbelags aus der Sterilisationskammer, in vielen Fällen ohne Abwarten einer längeren Einwirkzeit, unmittelbar nach beendetem Einströmen erfolgen. Das Trocknen der innerhalb der Sterilisationskammer befindlichen und mit Kondensat belegten Oberflächen erfolgt dabei durch bloßes Evakuieren der Sterilisationskammer auf einen Druck unterhalb von 10 mb, vorzugsweise unterhalb von 1 mb. Durch die dabei auftretende Druckdifferenz zwischen dem Innendruck innerhalb der Tubs und dem Druck in der Sterilisations-kammer wird die Luftströmung aus den Tubs heraus verstärkt, so dass auch weiterhin praktisch kein Dampfgemisch durch die Abdeckung hindurch in die Tubs hinein eindringen kann.

Bei einer anderen Variante des erfindungsgemäßen Verfahrens kann man sich den Umstand zunutze machen, dass die im Sub-µm-Bereich liegenden Porengrößen der Abdeckung verhindern, dass selbst kleinste Kondensattropfen die Poren durchdringen. Dank der schlagartigen Kondensatbildung, die mit sehr schnell anwachsenden und auch zusammenwachsenden Tröpfchen beginnt, findet durch das sich auch auf den Poren ablagernde Kondensat eine Art Dichtwirkung statt, so dass praktisch auch kein Dampf in nennenswertem Umfang durch die Poren hindurchdringen kann. Auch wenn eine solche Abdichtung durch das Kondensat keineswegs perfekt ist, ist sie dennoch gut genug, um das Eindringen von Wasserstoffperoxid in das Tub für eine Zeitspanne von einigen Sekunden weitestgehend zu unterbinden, zumindest aber zu verlangsamen. Diese Abdichtungswirkung ist für Zeitspannen von 2 bis 4 Sekunden ab Einströmbeginn des Dampfgemisches, bei geringeren Anforderungen auch für Zeitspannen bis 14 Sekunden, technisch nutzbar. Bei geeigneter Qualität der Abdeckung kann also nahezu kein Wasserstoffperoxid in das Tub eindringen, selbst wenn der Druck in der Sterilisationskammer während der Kondensatbildung über dem Druck im Inneren des Tubs liegt.

Als noch "zulässige Menge" von durch die Abdeckung hindurch auf die inneren Oberflächen von Spritzenkörpern oder die Oberflächen von sonstigen Gegenständen gelangendes Wasserstoffperoxid wird im Rahmen dieser Erfindung eine so genannte Wasserstoffperoxid-Restmenge angesehen, die, beispielsweise gemessen am Füllvolumen von Spritzenkörpern, 1,0 ppm (parts per million) nicht überschreitet und vorzugsweise sogar erheblich unter 0,5 ppm liegen sollte.

Unter dem Gesichtspunkt einer Verkürzung der Prozesszeit lassen sich vorteilhaft beide angesprochenen Verfahrensvarianten kombinieren. So ist es zweckmäßig, den Prozess so auszulegen, dass der Innendruck der Tubs zu Beginn des Dampfeinströmens noch deutlich höher liegt als der Druck in der Sterilisationskammer außerhalb der Tubs, dass aber anschließend der beim Einströmen sich aufbauende Dampfdruck der Sterilisationskammer umgekehrt den Innendruck der Tubs übersteigt, ohne auf Grund der erläuterten. Dichtwirkung des Kondensats zu schaden.

Eine weitere vorteilhafte Ausgestaltung ergibt sich, wenn der eigentliche Prozess des Nachsterilisierens, nämlich die Kondensataufbringung mit anschließendem Kondensatabziehen, wenigstens einmal wiederholt wird. Dabei kann insbesondere vorgesehen sein, dass zwischen dem Ende des Kondensatabziehens des ersten Sterilisationsvorganges und dem Beginn des Kondensataufbringens des nachfolgenden Sterilisationsvorganges die Auflagepunkte des Tubs auf seiner Unterlage vollständig verändert oder verschoben werden, so dass die während des ersten Sterilisationsvorganges eventuell durch die Unterlage abgedeckten Stellen des Tubs im nachfolgenden Sterilisationsvorgang für die Kondensataufbringung frei liegen. Hierdurch wird gewährleistet, dass lückenlos die gesamte außenliegende Oberfläche des Tubs wenigstens einmal mit Kondensat beaufschlagt und dadurch nachsterilisiert wird.

Sofern der eigentliche Prozess des Nachsterilisierens wiederholt wird, ist es im Prinzip ausreichend, wenn das Entfernen des im vorhergehenden Sterilisationsvorgang aufgebrachten Kondensatbelages durch Evakuieren der Sterilisationskammer nur bis zu einem Druck erfolgt, der unter dem Dampfdruck von Wasser bei der gegebenen Temperatur der Sterilisationskammer liegt, beispielsweise bis 70 mb im Falle einer beispielhaften Temperatur der Sterilisationskammer von 40°C. Zur Verringerung der Menge von eventuell in die Tubs eindringendem Wasserstoffperoxid ist es jedoch vorteilhafter, bis unter den Dampfdruck der verwendeten wässrigen Wasserstoffperoxidlösung bei der gegebenen Temperatur der Sterilisationskammer zu evakuieren, beim genannten Beispiel also - bei einer angenommenen Konzentration des eingeströmten Dampfgemisches von 50 Gewichtsprozent - auf unter 42 mb. Noch vorteilhafter sollte jedoch unter den Dampfdruck reinen Wasserstoffperoxids bei der gegebenen Temperatur der Sterilisationskammer evakuiert werden, bei einer Temperatur von 40°C der Sterilisationskammer somit unter 7 mb.

Weitere Vorteile und Merkmale der Erfindung ergeben sich aus der nachfolgenden Beschreibung eines Ausführungsbeispiels.

Es zeigen:
Figur 1 einen Querschnitt durch einen Spritzenkörper enthaltenden, abgedeckten und umverpackten Transportbehälter,
Figur 2 in schematischer Darstellung eine als Schleuse ausgebildete evakuierbare Sterilisationskammer zum Sterilisieren der Außenseite der von der Umverpackung befreiten Transportbehälter.

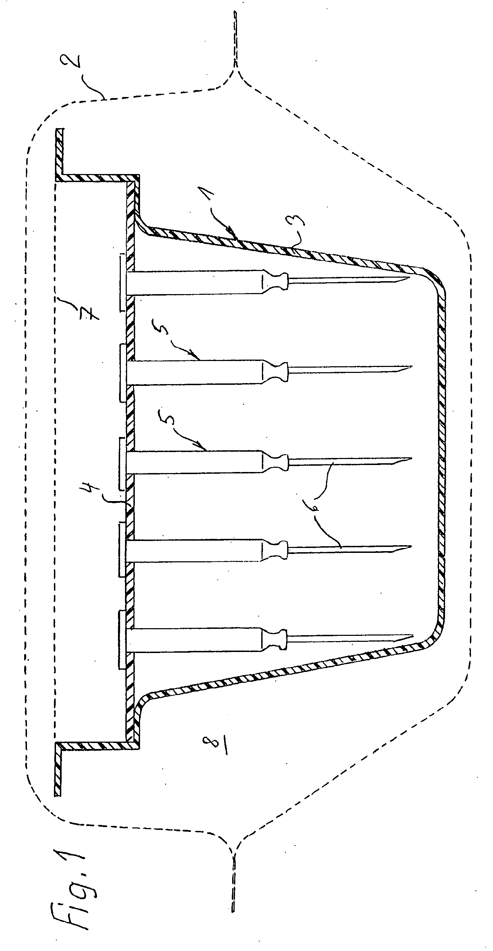

Der in der Figur 1 im Schnitt dargestellte Transportbehälter 1, ein so genanntes Tub, ist mit einer gestrichelt dargestellten Umverpackung 2 versehen, welche keimdicht, jedoch gasdurchlässig ist. Die Umverpackung 2 kann auch mehrschichtig sein, beispielsweise aus zwei gleichartigen, übereinander angeordneten Folien bestehen. Der Transportbehälter 1 selbst besteht aus einer Kunststoffwanne 3, in welche ein gelochtes Tablett 4, ein so genanntes Nest, eingesetzt ist. Darin ist eine Anzahl von Spritzenkörpern 5 eingehängt, die bereits mit eingeklebten Injektionsnadeln 6 und nicht dargestellten Nadelverschlusskappen sowie Nadelschutzkappen versehen sind. Diese Spritzenkörper 5 sind bereits sterilisiert worden und müssen später noch befüllt und mit einem Druckkolben verschlossen werden. Die Kunststoffwanne 3 ist auf ihrer Oberseite mit einer zwar keimdichten, aber gasdurchlässigen folienartigen Abdeckung 7 versiegelt. Das Innere 8 der Umverpackung 2 ist beim Sterilisieren der Spritzenkörper 5 mit sterilisiert worden.

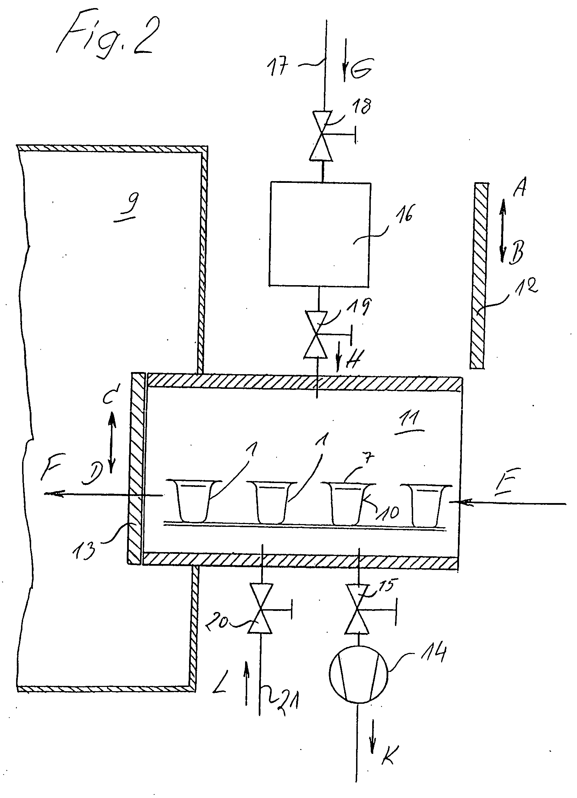

In Figur 2 ist schematisch ein steriler Reinraum 9 einer nicht dargestellten Füllvorrichtung angedeutet, in welchen die bereits sterilisierten Spritzenkörper 5 rekontaminationsfrei eingeführt werden müssen. Zu diesem Zwecke ist eine als Schleuse dienende Sterilisationskammer 11 vorgesehen, in welcher ausschließlich die Außenseite 10 der Transportbehälter 1, nachdem die Umverpackungen 2 abgenommen sind, nochmals sterilisiert werden.

Zu Beginn eines Zyklus ist die Sterilisationskammer 11 leer und die entsprechend den Bewegungsrichtungen A und B bewegbare Beladetür 12 geöffnet. Die Entladetür 13 ist hingegen geschlossen, damit eine Kontamination des Reinraumes 9 durch die Sterilisationskammer 11 hindurch vermieden wird. Die Bewegungsrichtungen der Entladetür 13 sind mit C und D bezeichnet.

Die der Umverpackung 2 entledigten, aber noch mit ihren Abdeckungen.7 verschlossenen Transportbehälter 1 werden nun in Einführrichtung E in die Sterilisationskammer 11 eingebracht, worauf die Beladetür 12 geschlossen wird.

Zu diesem Zeitpunkt, unmittelbar zu Beginn des Vorevakuierens, befindet sich noch Luft mit Atmosphärendruck innerhalb der Transportbehälter 1. Die keimdichte, aber gasdurchlässige Abdeckung 7 weist einen erheblichen Strömungswiderstand auf, was bedeutet, dass die Luft, in evakuierter Umgebung, nur langsam durch die Abdeckung 7 hindurch.nach außen strömen kann. Über einen Pumpstand 14 wird nun so rasch vorevakuiert, dass der Druck in der Sterilisationskammer 11 zunächst sehr viel schneller sinkt als der Druck innerhalb der Transportbehälter 1.

Sobald nun in der Sterilisationskammer 11 der gewünschte Vorevakuierungsdruck erreicht ist, wird das Abpumpventil 15 zum Pumpstand 14 hin geschlossen und unmittelbar darauf, nahezu gleichzeitig, ein Dampfventil 19 zu einem Verdampfer 16 geöffnet. Diesem Verdampfer 16 wird in Zulaufrichtung G über eine Zuleitung 17 und ein Zulaufventil 18 eine Wasserstoffperoxid enthaltende wässrige Lösung geeigneter Konzentration zugeführt, wonach im Verdampfer 16 ein aus Wasserdampf und Wasserstoffperoxiddampf bestehendes überhitztes Dampfgemisch erzeugt wird. Nach Öffnen des Dampfventils 19 kann dann in Dampfzuführrichtung H das Dampfgemisch ohne jeglichen Trägergasstrom, ausschließlich getrieben durch die Druckdifferenz zwischen dem Verdampfer 16 und der vorevakuierten Sterilisationskammer 11, in die Sterilisationskammer 11 einströmen. Dabei erfolgt in der bereits beschriebenen Weise ein Sterilisieren lediglich der Außenseiten 10 der einzelnen Transportbehälter 1. In dieser Prozessphase ist der Druck im Innern der Transportbehälter 1 kleiner als in der Sterilisationskammer 11 außerhalb der Transportbehälter 1.

Da die Abtötung der Keime unmittelbar im Augenblick der Kondensatbildung erfolgt, kann das Entfernen des Kondensatbelags aus der Sterilisationskammer 11, ohne Abwarten einer Einwirkzeit, unmittelbar nach beendetem Einströmen des Dampfgemisches und Schließen des Dampfventils 19 erfolgen. Dazu wird lediglich nochmals das Abpumpventil 15 geöffnet. Hierdurch wird der Druck in der Sterilisationskammer 11 sehr rasch verringert, und zwar so weit, dass dieser unterhalb der Dampfdrücke der beiden Komponenten des Kondensatbelags liegt, wobei dieser verdampfen und vom Pumpstand 14 aus der Sterilisationskammer 11 heraus abgesaugt werden kann. Nach beendeter Trocknung wird das Abpumpventil 15 geschlossen und ein Flutventil 20 geöffnet, so dass steriles Flutgas, im Regelfall Sterilluft, über eine Zuleitung 21 in Zuführrichtung L in die Sterilisationskammer 11 einströmen kann, bis der Druck in der Sterilisationskammer 11 dem Druck im sterilen Reinraum 9 entspricht. Zur Entladung wird dann die Entladetür 13 geöffnet, wodurch die an ihren Außenseiten 10 nachsterilisierten Transportbehälter 1 in Ausführrichtung F in den sterilen Reinraum 9 eingebracht werden. Dabei bleibt die Beladetür 12 so lange geschlossen, bis die Entladetür 13 nach der Entnahme der Transportbehälter 1 wieder vollständig geschlossen ist und den Reinraum 9 abdichtet. Wenn anschließend die Beladetür 12 wieder geöffnet wird, ist der Anfangszustand wieder erreicht.

Wie aus dem Verfahrensablauf ersichtlich, ist besonderes Augenmerk auf die Phase der Vorevakuierung zu legen. So sollte durch sorgfältige Steuerung des Druckverlaufs in der Sterilisationskammer 11 darauf geachtet werden, dass der auftretende Druckunterschied außerhalb und innerhalb der Transportbehälter 1 nicht derart groß wird, dass etwa die Befestigungen der Abdeckungen 7 beschädigt werden. Durch den entstehenden Druckunterschied können sich nämlich die folienartigen Abdeckungen 7 verformen, was diesen selbst jedoch nicht schadet.

Sollte während des Vorevakuierens, beispielsweise aufgrund einer mangelhaften Befestigung der Abdeckung 7 auf der Kunststoffwanne 3, sich die Abdeckung 7 teilweise von der Kunststoffwanne 3 lösen, hätte dies eine unakzeptabel hohe Wasserstoffperoxid-Restmenge auf den im Transportbehälter 1 befindlichen Gegenständen, beispielsweise den Spritzenkörpern 5 zur Folge, da ja beim Einströmen des Dampfgemisches in die Sterilisationskammer 11 dieses direkt zu den Gegenständen gelangen könnte. Weil beim Vorevakuieren nun der Druck im Transportbehälter 1 zunächst noch deutlich über dem Druck in der Sterilisationskammer 11 außerhalb der Transportbehälter 1 liegt, gibt es bei einem etwaigen Aufreißen der Abdeckung 7 einen kurzen Druckstoß, der sich mit einem an der Sterilisationskammer 11 angebrachten Druckaufnehmer registrieren lässt. Ein solcher Druckstoß kann von der Steuereinrichtung der Anlage erfasst werden, so dass eine Warnmeldung ausgegeben werden kann, dass der betroffene Transportbehälter 1 oder eventuell die gesamte Beladung der Sterilisationskammer 11 dieses Zyklus aussortiert werden muss.

Sollte ein in die Sterilisationskammer 11 eingebrachter Transportbehälter 1 oder dessen Abdeckung 7 bereits beschädigt sein, bevor das Nachsterilisieren stattfindet, so kann dies ebenfalls während des ablaufenden Prozesses registriert werden. Dies ist ein großer Vorzug der Erfindung gegenüber dem Stand der Technik.

Solange während des Vorevakuierens oder des weiteren Evakuierens zum Entfernen des Kondensatbelages der Druck im Innern der Transportbehälter 1 über dem Druck der Sterilisationskammer 11 liegt, kann sich die flexible Abdeckung 7 nach außen aufwölben. Ist eine Abdeckung 7 oder ein Transportbehälter 1 beschädigt, so dass durch diese offene Stelle das Gas schneller entweichen kann, als dies aufgrund des Strömungswiderstandes der Abdeckung 7 der Fall wäre, findet nur ein vermindertes oder kein Aufwölben statt.

## Patentansprüche

1. Verfahren zum Handhaben eines Verpackungs-Transportsystems, bei welchem kontaminierte Gegenstände in einer kontaminierten Verpackung keimdicht, aber gasdurchlässig verpackt und in diesem Zustand sterilisiert werden und die Verpackung in diesem Zustand, gegebenenfalls nach Entfernen einiger Verpackungsteile, in einer Schleuse auf ihrer Außenseite erneut sterilisiert und in einen sterilen Reinraum eingeführt wird, **dadurch gekennzeichnet, dass** das erneute Sterilisieren der Außenseite der Verpackung in einer als Schleuse dienenden evakuierbaren Sterilisationskammer durchgeführt wird, wobei ein aus Wasserdampf und Wasserstoffperoxiddampf bestehendes Dampfgemisch durch Vorevakuieren der Sterilisationskammer schlagartig als Kondensatbelag auf die Außenseite der Verpackung aufgebracht wird und unmittelbar danach der Kondensatbelag und das nicht kondensierte Dampfgemisch durch weiteres Evakuieren aus der Sterilisationskammer entfernt werden, so dass weder das Dampfgemisch noch der Kondensatbelag in unzulässiger Menge durch die Verpackung hindurch zu den Gegenständen gelangen.

2. Verfahren zum rekontaminationsfreien Einführen von bereits sterilisierten, gegebenenfalls mit Injektionsnadeln versehenen Spritzenkörpern in eine von einem sterilen Reinraum umgebene Füllvorrichtung zum Befüllen und Verschließen der Spritzenkörper, bei welchem Spritzenkörper enthaltende sterilisierte Transportbehälter (so genannte Tubs), die mit einer keimdichten, aber gasdurch-lässigen Abdeckung versehen und zusätzlich von einer ebenfalls keimdichten und gasdurch-lässigen Umverpackung umschlossen sind, zunächst von der Umverpackung befreit und danach, während sie noch mit der Abdeckung versehen sind, auf ihrer Außenseite erneut sterilisiert und in den Reinraum eingeführt werden, **dadurch gekennzeichnet, dass** das erneute Sterilisieren der Außenseite der Transportbehälter in einer als Schleuse dienenden evakuierbaren Sterilisationskammer durchgeführt wird, wobei ein aus Wasserdampf und Wasserstoffperoxiddampf bestehendes Dampfgemisch durch Vorevakuieren der Sterilisationskammer schlagartig als Kondensatbelag auf die Außenseite der Transportbehälter aufgebracht wird und unmittelbar danach der Kondensatbelag und das nicht kondensierte Dampfgemisch durch weiteres Evakuieren aus der Sterilisationskammer entfernt werden, so dass weder das Dampfgemisch noch der Kondensatbelag in unzulässiger Menge durch die Abdeckung hindurch zu den Spritzenkörpern gelangen.

3. Verfahren nach Anspruch 1 oder 2, **dadurch gekennzeichnet, dass** das Dampfgemisch ohne Trägergasstrom ausschließlich infolge Druckdifferenz in die vorevakuierte Sterilisationskammer eingeführt wird.

4. Verfahren nach einem der Ansprüche 1 bis 3, **dadurch gekennzeichnet, dass** die Geschwindigkeit des Vorevakuierens auf den Strömungswiderstand der gasdurchlässigen Abdeckung der Transportbehälter abgestimmt wird.

5. Verfahren nach einem der Ansprüche 1 bis 4, **dadurch gekennzeichnet, dass** der Kondensatbelag ohne Abwarten einer Einwirkzeit unmittelbar nach beendetem Einströmen des Dampfgemisches wieder aus der Sterilisationskammer entfernt wird.

6. Verfahren nach einem der Ansprüche 1 bis 5, **dadurch gekennzeichnet, dass** das Entfernen des Kondensatbelages durch Evakuieren der Sterilisationskammer auf einen Druck unterhalb 10 mb erfolgt.

7. Verfahren nach Anspruch 6, **dadurch gekennzeichnet, dass** das Entfernen des Kondensatbelages durch Evakuieren der Sterilisationskammer auf einen Druck unterhalb 1 mb erfolgt.

8. Verfahren nach einem der Ansprüche 1 bis 7, **dadurch gekennzeichnet, dass** nach dem Entfernen des Kondensatbelages die Sterilisationskammer mit Sterilluft geflutet wird.

9. Verfahren nach einem der Ansprüche 1 bis 8, **dadurch gekennzeichnet, dass** der Kondensatbelag aufgebracht wird, bevor der Innendruck der Verpackung bzw. der Transportbehälter sich an den Druck der Sterilisationskammer angeglichen hat.

10. Verfahren nach einem der Ansprüche 1 bis 9, **dadurch gekennzeichnet, dass** als unzulässige Menge eine Wasserstoffperoxid-Restmenge angesehen wird, die einen Wert von 1,0 ppm überschreitet.

11. Verfahren nach Anspruch 10, **dadurch gekennzeichnet, dass** die Wasserstoffperoxid-Restmenge einen Wert von 0,5 ppm nicht überschreitet.

12. Verfahren nach Anspruch 11, **dadurch gekennzeichnet, dass** die Wasserstoffperoxid-Restmenge einen Wert von 0,5 ppm wesentlich unterschreitet.

13. Verfahren nach einem der Ansprüche 1 bis 12, **dadurch gekennzeichnet, dass** vom Beginn des Einströmens des Dampfgemisches bis zum Beginn des weiteren Evakuierens eine Zeitdauer von maximal 14 Sekunden vorgesehen ist.

14. Verfahren nach Anspruch 13 , **dadurch gekennzeichnet, dass** die Zeitdauer maximal 4 Sekunden beträgt.

15. Verfahren nach Anspruch 14, **dadurch gekennzeichnet, dass** die Zeitdauer maximal 2 Sekunden beträgt.

16. Verfahren nach einem der vorhergehenden Ansprüche, **dadurch gekennzeichnet, dass** das alleinige Sterilisieren der Außenseite der Verpackung bzw. der Transportbehälter durch die Auswahl der Porigkeit der Abdeckung (7) gesteuert wird.

17. Verfahren nach einem der vorhergehenden Ansprüche, **dadurch gekennzeichnet, dass** der eigentliche, aus Kondensataufbringung und anschließendem Kondensatabziehen bestehende Prozess des Nachsterilisierens wenigstens einmal wiederhoit wird.

18. Verfahren nach Anspruch 17, **dadurch gekennzeichnet, dass** den Transportbehältern Auflagestellen einer Halterung oder Transporteinrichtung zugeordnet sind und dass vor einem Wiederholen des eigentlichen Prozesses des Nachsterilisierens die Auflagestellen verändert werden.

19. Verfahren nach Anspruch 17 oder 18, **dadurch gekennzeichnet, dass** bei einem Wiederholen des eigentlichen Prozesses des Nachsterilisierens das Entfernen des im vorangegangenen Sterilisationsvorgang aufgebrachten Kondensatbelags durch Evakuieren der Sterilisationskammer bis zu einem Druck erfolgt, der unter dem Dampfdruck von Wasser bei der gegebenen Temperatur der Sterilisationskammer liegt.

20. Verfahren nach Anspruch 19, **dadurch gekennzeichnet, dass** das Entfernen des Kondensatbelages durch Evakuieren der Sterilisationskammer bis zu einem Druck erfolgt, der unter dem Dampfdruck der verwendeten wässrigen Wasserstoffperoxidlösung bei der gegebenen Temperatur der Sterilisationskammer liegt.

21. Verfahren nach Anspruch 20, **dadurch gekennzeichnet, dass** das Entfernen des Kondensatbelages durch Evakuieren der Sterilisationskammer bis zu einem Druck erfolgt, der unter dem Dampfdruck von reinem Wasserstoffperoxid bei der gegebenen Temperatur der Sterilisationskammer liegt.

22. Verfahren nach einem der vorhergehenden Ansprüche, **dadurch gekennzeichnet, dass** eine auftretende Undichtigkeit der Transportbehälter durch Analyse des Druckverlaufs in der Sterilisationskammer detektiert wird.

23. Verfahren nach Anspruch 22, **dadurch gekennzeichnet, dass** eine auftretende Undichtigkeit während des Vorevakuierens detektiert wird.

24. Verfahren nach Anspruch 22 oder 23, **dadurch gekennzeichnet, dass** eine auftretende Undichtigkeit während des Evakuierens zum Entfernen des Kondensatbelages detektiert wird.

25. Verfahren nach einem der vorhergehenden Ansprüche, **dadurch gekennzeichnet, dass** eine bereits beim Einführen der Transportbehälter in die Sterilisationskammer vorhandene Undichtigkeit durch Kontrollieren des Aufwölbens bzw. Nichtaufwölbens der Abdeckung detektiert wird.

26. Verfahren nach Anspruch 25, **dadurch gekennzeichnet, dass** das Detektieren während des Vorevakuierens stattfindet.

27. Verfahren nach Anspruch 25 oder 26, **dadurch gekennzeichnet, dass** das Detektieren während des Evakuierens zum Entfernen des Kondensatbelages stattfindet.
